# EUROPEAN PATENT APPLICATION

(11) **EP 4 596 545 A1**
(43) Date of publication of application: **06.08.2025**
(21) Application number: 23870876.2
(22) Date of filing: 26.09.2023
(51) Int. Cl.: C07D 471/04, C07D 487/04, C08G 73/10, C08J 5/18, H01M 10/613

(54) **DIAMINE COMPOUND, POLYIMIDE ACID, POLYIMIDE, AND PREPARATION METHOD THEREFOR AND USE THEREOF**

(30) Priority: 30.09.2022 CN 202211212701
(71) Applicant: BYD Company Limited, Shenzhen, Guangdong 518118 (CN)
(72) Inventor: HE, Junpeng, Shenzhen, Guangdong 518118 (CN); DUAN, Pingping, Shenzhen, Guangdong 518118 (CN); ZHANG, Min, Shenzhen, Guangdong 518118 (CN); WU, Hongsheng, Shenzhen, Guangdong 518118 (CN); YAO, Cheng, Shenzhen, Guangdong 518118 (CN)
(74) Representative: Mathys & Squire
(86) International application number: PCT/CN2023/121799
(87) International publication number: WO 2024/067649

(57) **Abstract**

The present application provides a diamine compound, a polyimide acid, a polyimide, and a preparation method therefor and use thereof. The diamine compound has a chemical structural formula represented by formula (I), 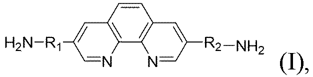 wherein R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene.

## Description

### CROSS-REFERENCE TO RELATED APPLICATIONS

The present disclosure claims priority to Chinese Patent Application No. 202211212701.2 filed on September 30, 2022 and entitled "diamine compound, polyimide acid, polyimide, and preparation methods therefor and use thereof", which is incorporated herein by reference in its entirety.

### FIELD

The present disclosure relates to the technical field of materials, and specifically to a diamine compound, a polyimide acid, a polyimide, and preparation methods therefor and use thereof.

### BACKGROUND

With the continuous development of electronic devices, polyimide films with various advantages have become a key material receiving great interest from manufacturers. However, the bonding strength between polyimide and metal members is poor at present, which limits the use and development of polyimide. Therefore, there is a need to improve the performance of polyimide.

### SUMMARY

In view of this, the present disclosure provides a diamine compound, a polyimide acid, a polyimide, and a preparation method therefor and use thereof. The diamine compound, the polyimide acid and the polyimide have a 1,10-phenanthrolinyl group, which can coordinate with a metal atom to form a strong coordination bond, thus improving the bonding strength between polyimide and metal members, and promoting the use of polyimide.

In a first aspect, the present disclosure provides a diamine compound. The diamine compound has a chemical structural formula represented by Formula (I): 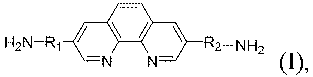 where R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene.

The diamine compound provided in the present disclosure has a 1,10-phenanthrolinyl group, and the diamine compound can be used as a raw material for preparing a polyimide, so that the polyimide also has a 1,10-phenanthrolinyl group, to ensure the bonding strength between the polyimide and a metal member.

In an embodiment of the present disclosure, the substituted or unsubstituted alkylene is a substituted or unsubstituted C1-C8 alkylene;
the substituted or unsubstituted alkenylene is a substituted or unsubstituted C2-C8 alkenylene;
the substituted or unsubstituted alkynylene is a substituted or unsubstituted C2-C8 alkynylene;
the substituted or unsubstituted arylene is a substituted or unsubstituted C6-C30 arylene;
the substituted or unsubstituted arylene is a substituted or unsubstituted C7-C40 arylenealkyl;
the substituted or unsubstituted heteroarylene is a substituted or unsubstituted C2-C30 heteroarylene;
the substituted or unsubstituted arylene is a substituted or unsubstituted C3-C40 heteroarylenealkyl; and
the substituted or unsubstituted alicylidene is a substituted or unsubstituted C3-C30 alicylidene.

In an embodiment of the present disclosure, R₁ and R₂ are independently selected from a single bond, substituted or unsubstituted C6-C30 arylene, or substituted or unsubstituted C7-C40 arylenealkyl.

In an embodiment of the present disclosure, the diamine compound comprises one of the compounds represented by Formulas (I-1) to (I-4):

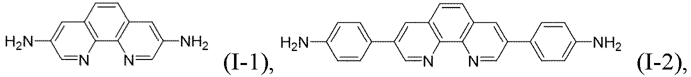

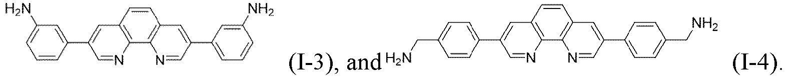

In a second aspect, the present disclosure provides a method for preparing a diamine compound. The method comprises the following steps.

A first reactant is provided. The first reactant has a chemical structural formula represented by Formula (II), where R₃ and R₄ are independently selected from chlorine atom, bromine atom, iodine atom, or astatine atom,

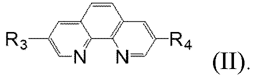

A second reactant is provided. The second reactant has a chemical structural formula represented by Formula (III), where R₅ is selected from hydrogen, an alkyl substituted with boric acid or a borate ester, an alkenyl substituted with boric acid or a borate ester, an alkynyl substituted with boric acid or a borate ester, an aryl substituted with boric acid or a borate ester, an aralkyl substituted with boric acid or a borate ester, a heteroaryl substituted with boric acid or a borate ester, a heteroarylalkyl substituted with boric acid or a borate ester, or an alicylyl substituted with boric acid or a borate ester,

H₂N-R₅ (III).

The first reactant and the second reactant are mixed under basic conditions to form a reaction solution, and reacted to obtain the diamine compound. The diamine compound has a chemical structural formula represented by Formula (I), where R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene,

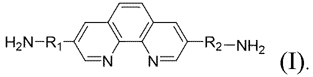

The method for preparing a diamine compound in the present disclosure is simple and convenient in operation, by which large-scale production of the diamine compound can be achieved, to promote the production and use of the polyimide.

In an embodiment of the present disclosure, the reaction solution further comprises a catalyst and a catalyst ligand.

The catalyst comprises a copper-based catalyst and a palladium-based catalyst. The copper-based catalyst comprises cuprous oxide, and the palladium-based catalyst comprises at least one of bis(3,5,3',5'-dimethoxydibenzylideneacetone) palladium, bis(tri-tert-butyl) palladium, tris(dibenzylideneacetone) palladium, palladium chloride, palladium acetate, tetrakis(triphenylphosphine) palladium, and bis(tri-tert-butylphosphine) palladium.

The catalyst ligand comprises at least one of N,N'-dimethyl ethylenediamine, triphenylphosphine, 2-dicyclohexylphosphino-2',4',6'- triisopropylbiphenyl, 4,5-bis(diphenylphosphino)-9,9-dimethyl xanthene, triphenylphosphine oxide, and tris(o-methylphenyl) phosphine.

The molar ratio of the first reactant, the second reactant, the catalyst, and the catalyst ligand is 1: (2-50): (0.005-0.2): (0.005-0.5).

The reaction solution further comprises an alkaline substance, and the alkaline substance comprises at least one of potassium carbonate, sodium carbonate, cesium fluoride, sodium hydroxide, potassium hydroxide, and barium hydroxide.

The molar ratio of the first reactant, the second reactant, and the alkaline substance is 1: (2-50): (0.05-10).

The reaction temperature is 25°C to 180°C, and the time is 2 h to 72 h.

In an embodiment of the present disclosure, the reaction solution further comprises a solvent, and the solvent comprises water, ethylene glycol, and 1,4-dioxane, where the volume ratio of 1,4- dioxane to water is 2 to 10.

In an embodiment of the present disclosure, the reaction is carried out under an inert atmosphere.

In a third aspect, the present disclosure provides a polyimide acid. The polyimide acid comprises a repeat unit represented by Formula (IV): 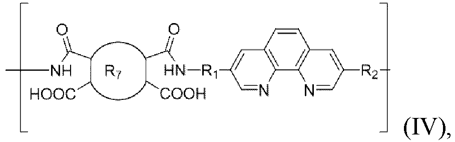 where R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R7 is selected from a substituted or unsubstituted arylene or substituted or unsubstituted heteroarylene.

The polyimide acid provided in the present disclosure has a 1,10-phenanthrolinyl group, which can form a chemical bond with a metal member, to improve the adhesion to the metal member, thus promoting the preparation of the polyimide and improving the bonding strength between the polyimide and the metal member.

In an embodiment of the present disclosure, the polyimide acid has a chemical structural formula represented by Formula (V): 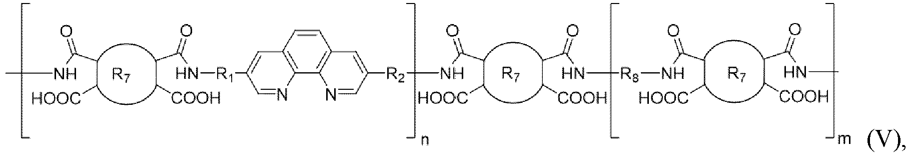 where R₈ is selected from a substituted or unsubstituted alkylene, substituted or unsubstituted arylene, or substituted or unsubstituted heteroarylene, n is 5 to 2000, and m is 0 to 1000.

In a fourth aspect, the present disclosure provides a method for preparing a polyimide acid, which comprises the following steps. A first diamine compound is mixed with a dianhydride to form a mixed solution, and reacted to obtain the polyimide acid. The first diamine compound is a diamine compound according to the first aspect or a diamine compound prepared through the preparation method according to the second aspect. The polyimide acid comprises a repeat unit represented by Formula (IV), where R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R₇ is selected from a substituted or unsubstituted arylene or substituted or unsubstituted heteroarylene,

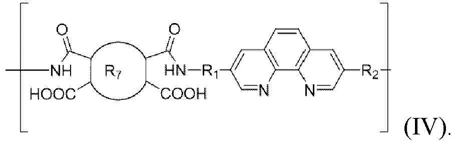

The method for preparing a polyimide acid provided in the present disclosure is simple and convenient in operation, by which large-scale production of the polyimide acid can be achieved, to promote the preparation of the polyimide acid.

In an embodiment of the present disclosure, the molar ratio of the first diamine compound to the dianhydride is 0.9 to 1.1.

The dianhydride comprises at least one of pyromellitic dianhydride, 2,3,3',4'-diphenyl ether tetracarboxylic dianhydride, 3,3',4,4'-benzophenone tetracarboxylic dianhydride, 3,3',4,4'-diphenyl sulfone tetracarboxylic dianhydride, 2,2-bis(3,4-dicarboxyphenyl)hexafluoropropane dianhydride, 4,4'-bis(3,4-dicarboxyphenoxy)diphenyl ether dianhydride, 4,4'-bis(3,4-dicarboxyphenoxy)benzophenone dianhydride, 4,4'-bis(3,4-dicarboxyphenoxy)diphenylmethane dianhydride, and 4,4'-bis(3,4-dicarboxyphenoxy)diphenyl sulfone dianhydride.

The mixed solution further comprises a second diamine compound, and the second diamine compound comprises at least one of 4,4'-diaminodiphenyl ether, m-phenylenediamine, p-phenylenediamine, 4,4'-diaminodiphenyl sulfone, 1,3-diamino-2-methylpropane, N,N-bis(4-aminophenyl)-1,4-phenylenediamine, 9,9-bis(4-aminophenyl)fluorene, 1,2-diaminocyclohexane, and ethylenediamine.

The mixed solution further comprises a solvent, and the solvent comprises at least one of dimethyl sulfoxide, N-methylpyrrolidone, N,N-dimethylformamide, N,N-dimethylacetamide, and m-cresol.

The reaction temperature is 0°C to 100°C, and the time is 2 h to 12 h.

In a fifth aspect, the present disclosure provides a polyimide. The polyimide has a repeat unit represented by Formula (VI): 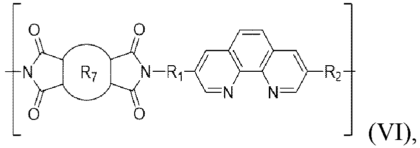 where R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R₇ is selected from a substituted or unsubstituted arylene or substituted or unsubstituted heteroarylene.

The polyimide provided in the present disclosure has a 1,10-phenanthrolinyl group, which can form a chemical bond with a metal member, to improve the adhesion of the polyimide to the metal member, and promote the use of the polyimide.

In an embodiment of the present disclosure, the polyimide has a chemical structural formula represented by Formula (VII): 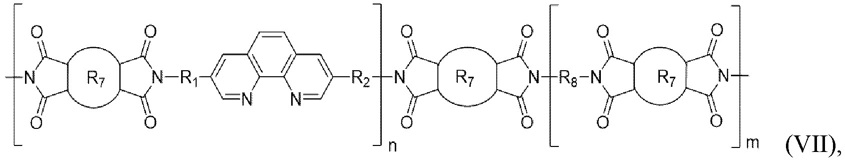 Where R₈ is selected from a substituted or unsubstituted alkylene, substituted or unsubstituted arylene, or substituted or unsubstituted heteroarylene, n is 5 to 2000, and m is 0 to 1000.

In a sixth aspect, the present disclosure provides a method for preparing a polyimide, which comprises the following steps. The polyimide acid according to the third aspect or the polyimide acid prepared by the preparation method according to the fourth aspect is imidized to obtain the polyimide. The polyimide has a repeat unit represented by Formula (VI), where R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R₇ is selected from a substituted or unsubstituted arylene or substituted or unsubstituted heteroarylene,

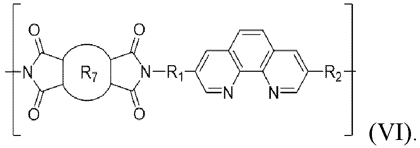

The method for preparing a polyimide provided in the present disclosure is simple and convenient in operation, by which large-scale production of the polyimide can be achieved, and a polyimide having a high bonding strength with a metal member can be prepared, thus promoting the use of the polyimide.

In a seventh aspect, the present disclosure provides a battery assembly, which comprises a heat dissipation component, a polyimide film arranged on a surface of the heat dissipation component, and a battery core arranged on a surface of the polyimide film. The polyimide film is made of the polyimide according to the fifth aspect or the polyimide prepared by the preparation method according to the sixth aspect.

In the battery assembly provided in the present disclosure, the heat dissipation component and the battery core are connected by the polyimide film. The polyimide film improves the bonding strength to the heat dissipation component, and the polyimide is insulating, to further ensure the safety of the battery assembly.

In an eighth aspect, the present disclosure provides an electronic device, which comprises the battery assembly according to the seventh aspect.

The battery assembly in the electronic device provided in the present disclosure has a high safety, thus promoting the use of the electronic device.

### BRIEF DESCRIPTION OF THE DRAWINGS

In order to describe the technical solutions according to the embodiments of the present disclosure or in the prior art more clearly, the drawings needed to be used in the embodiments or in the prior art will be described briefly below. The specific embodiments described herein are merely used to explain the present disclosure, and the present disclosure is not limited thereto.
FIG. 1 schematically shows the bonding of a polyimide to a metal atom provided in an embodiment of the present disclosure.
FIG. 2 is a schematic cross-sectional view of a battery assembly provided in an embodiment of the present disclosure.
FIG. 3 schematically shows the bonding of a polyimide film to a heat dissipation component provided in an embodiment of the present disclosure.

List of reference numerals:
10. battery assembly, 11. battery core, 12. polyimide film, 13. heat dissipation component

### DETAILED DESCRIPTION

The technical solution according to the embodiments of the present disclosure will be described clearly and fully below with reference to the accompanying drawings in the embodiments of the present disclosure. Apparently, the embodiments described are merely some, but not all of the embodiments of the present invention. All other embodiments obtained by a person of ordinary skill in the art based on the embodiments of the present disclosure without creative efforts shall fall within the protection scope of the present invention.

The present disclosure provides a diamine compound. The diamine compound has a chemical structural formula represented by Formula (I): 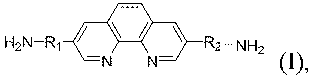 where R₁, and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene. The diamine compound provided in the present disclosure has a 1,10-phenanthrolinyl group (that is, 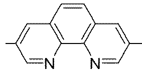 ). The diamine compound can be used as a raw material for preparing a polyimide, so that the prepared polyimide also has a 1,10-phenanthrolinyl group, to ensure the bonding strength between the polyimide and a metal member.

In the present disclosure, R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene. R₁ is selected from a single bond, alkylene, substituted alkylene, alkenylene, substituted alkenylene, alkynylene, substituted alkynylene, arylene, substituted arylene, arylenealkyl, substituted arylenealkyl, heteroarylene, substituted heteroarylene, heteroarylenealkyl, substituted heteroarylenealkyl, alicylidene, or substituted alicylidene. R₂ is selected from a single bond, alkylene, substituted alkylene, alkenylene, substituted alkenylene, alkynylene, substituted alkynylene, arylene, substituted arylene, arylenealkyl, substituted arylenealkyl, heteroarylene, substituted heteroarylene, heteroarylenealkyl, substituted heteroarylenealkyl, alicylidene, or substituted alicylidene.

In the present disclosure, the alkyl is a group obtained by removing one hydrogen atom from an alkane molecule, and may comprise a linear alkyl and a branched alkyl. Particularly, the alkyl may comprise, but is not limited to, at least one of methyl, ethyl, n-propyl, isopropyl, n-butyl, isobutyl, s-butyl, t-butyl, n-pentyl, 2-methylbutyl, 3-methylbutyl, 4-methylbutyl, 2,2-dimethylpropyl, n-hexyl, heptyl, 2-methylhexyl, 3-methylhexyl, 4-methylpentyl, 5-methylpentyl, 2-ethylbutyl, 3-ethylbutyl, heptyl, octyl, nonyl, and decyl. In an embodiment of the present disclosure, the substituted or unsubstituted alkyl may be a substituted or unsubstituted C₁-C₈ alkyl. That is, the alkyl has 1-8 carbon atom(s). Particularly, the alkyl has, without limitation, 1, 2, 3, 4, 5, 6, 7 or 8 carbon atoms.

In the present disclosure, the alkylene is a divalent saturated group formed by removing one hydrogen atom from the alkyl. Particularly, the alkylene may comprise, but is not limited to, at least one of -CH₂-, -CH₂CH₂-, -CH₂CH₂CH₂-, -CH₂CH₂CH₂CH₂-, - CH₂CH₂CH₂CH₂CH₂-, and -CH₂CH₂CH₂CH₂CH₂CH₂-. In an embodiment of the present disclosure, the substituted or unsubstituted alkylene is a substituted or unsubstituted C₁-C₈ alkylene. That is, the alkylene has 1 to 8 carbon atom(s). Particularly, the alkylene has, without limitation, 1, 2, 3, 4, 5, 6, 7 or 8 carbon atoms.

In the present disclosure, the alkenyl is a bivalent unsaturated hydrocarbon chain containing at least one double bond, and may comprise a linear alkenyl or a branched alkenyl. Particularly, the alkenyl may comprise, but is not limited to, at least one of ethenyl, propenyl, isopropenyl, butenyl, butadienyl, pentenyl, pentadienyl, hexenyl, hexadienyl, heptenyl, octenyl, nonenyl, and decenyl. In an embodiment of the present disclosure, the substituted or unsubstituted alkenyl is a substituted or unsubstituted C₂-C₈ alkenyl. That is, the alkenyl has 2 to 8 carbon atoms. Particularly, the alkenyl has, without limitation, 2, 3, 4, 5, 6, 7 or 8 carbon atoms.

In the present disclosure, the alkenylene is a divalent unsaturated group formed by removing one hydrogen atom from the alkenyl. Particularly, the alkenylene may comprise, but is not limited to, at least one of -CH=CH-, -CH=CHCH₂-, -CH₂CH=CH-, -CH=CHCH₂CH₂-, - CH₂CH₂CH=CH-, -CH₂CH=CHCH₂-, -CH=CH-CH=CH-, -CH=CHCH₂CH₂CH₂-, -CH=CH-CH=CH₂CH₂-, and -CH=CH₂CH₂CH=CH-. In an embodiment of the present disclosure, the substituted or unsubstituted alkenylene is a substituted or unsubstituted C₂-C₈ alkenylene. That is, the alkenylene has 2 to 8 carbon atoms. Particularly, the alkenylene has, without limitation, 2, 3, 4, 5, 6, 7 or 8 carbon atoms.

In the present disclosure, the alkynyl is a trivalent unsaturated hydrocarbon chain containing at least one triple bond, and may comprise a linear alkynyl or a branched alkynyl. Particularly, the alkynyl may comprise, but is not limited to, at least one of ethynyl, propynyl, butynyl, pentynyl, and hexynyl. In an embodiment of the present disclosure, the substituted or unsubstituted alkynyl is a substituted or unsubstituted C₂-C₈ alkynyl. That is, the alkynyl has 2 to 8 carbon atoms. Particularly, the alkynyl has, without limitation, 2, 3, 4, 5, 6, 7 or 8 carbon atoms.

In the present disclosure, the alkynylene is a divalent unsaturated group formed by removing one hydrogen atom from the alkynyl. Particularly, the alkynylene may comprise, but is not limited to, at least one of -C≡C-, -C≡CCH₂-, -CH₂C≡C-, -C≡CCH₂CH₂-, -CH₂C≡CCH₂-, - CH₂CH₂C≡C-, -C≡C-C≡C-, -C≡CCH₂CH₂CH₂-, -CH₂C≡CCH₂CH₂-, -CH₂CH₂C≡CCH₂-, - CH₂C≡C-C≡C-CH₂-, -C≡CCH₂CH₂CH₂CH₂-, -CH₂C≡CCH₂CH₂CH₂-, -CH₂CH₂C≡CCH₂CH₂-, - CH₂CH₂CH₂C≡CCH₂-, and -CH₂CH₂CH₂CH₂C≡C-. In an embodiment of the present disclosure, the substituted or unsubstituted alkynylene is a substituted or unsubstituted C₂-C₈ alkynylene. That is, the alkynylene has 2 to 8 carbon atoms. Particularly, the alkynylene has, without limitation, 2, 3, 4, 5, 6, 7 or 8 carbon atoms.

In the present disclosure, the aryl is an aromatic group. Particularly, the aryl may comprise, but is not limited to, at least one of phenyl, naphthalenyl, anthracenyl, tetracenyl, pentacenyl, and tetrahydronaphthalenyl. In an embodiment of the present disclosure, the substituted or unsubstituted aryl is a substituted or unsubstituted C₆-C₃₀ aryl. That is, the aryl has 6 to 30 carbon atoms. Particularly, the aryl has, without limitation, 6, 10, 12, 14, 18, 22, 24, 26 or 30 carbon atoms.

In the present disclosure, the arylene is a divalent aromatic group. Particularly, the arylene may comprise, but is not limited to, at least one of phenylene, naphthalenylene, anthracenylene, tetracenylene, pentacenylene, and tetrahydronaphthalenylene. In an embodiment of the present disclosure, the substituted or unsubstituted arylene is a substituted or unsubstituted C₆-C₃₀ arylene. That is, the arylene has 6 to 30 carbon atoms. Particularly, the arylene has, without limitation, 6, 10, 12, 14, 18, 22, 24, 26 or 30 carbon atoms.

In the present disclosure, the arylenealkyl is a combined group formed by linking an arylene to an alkylene. Particularly, the arylenealkyl may comprise, but is not limited to, at least one of 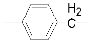 , and 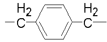 . In an embodiment of the present disclosure, the substituted or unsubstituted arylenealkyl is a substituted or unsubstituted C₇-C₄₀ arylenealkyl. That is, the arylenealkyl has 7 to 40 carbon atoms. Particularly, the arylenealkyl has, without limitation, 7, 8, 9, 10, 15, 18, 20, 25, 26, 30, 32, 37 or 40 carbon atoms.

In the present disclosure, the heteroaryl is an aryl having at least one oxygen, sulfur or nitrogen atom. Particularly, the heteroaryl may comprise, but is not limited to, at least one of pyridyl, furyl, thienyl, indolyl, quinolinyl, imidazolinyl, and thiazolyl. In an embodiment of the present disclosure, the substituted or unsubstituted heteroaryl is a substituted or unsubstituted C₂-C₃₀ heteroaryl. That is, the heteroaryl has 2 to 30 carbon atoms. Particularly, the heteroaryl has, without limitation, 3, 5, 8, 12, 17, 20, 25, 28 or 30 carbon atoms.

In the present disclosure, the heteroarylene is a divalent heteroaryl. Particularly, the heteroarylene may comprise, but is not limited to, at least one of pyridylene, furylene, thienylene, indolylene, quinolinylene, imidazolinylene, and thiazolylene. In an embodiment of the present disclosure, the substituted or unsubstituted heteroarylene is a substituted or unsubstituted C₂-C₃₀ heteroarylene. That is, the heteroarylene has 2 to 30 carbon atoms. Particularly, the heteroarylene has, without limitation, 3, 5, 8, 12, 17, 20, 25, 28 or 30 carbon atoms.

In the present disclosure, the heteroarylenealkyl is a combined group formed by linking a heteroarylene to an alkylene. Particularly, the heteroarylenealkyl may comprise, but is not limited to, at least one of 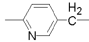 , and 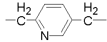 . In an embodiment of the present disclosure, the substituted or unsubstituted heteroarylenealkyl is a substituted or unsubstituted C₃-C₄₀ heteroarylenealkyl. That is, the heteroarylenealkyl has 2 to 40 carbon atoms. Particularly, the heteroarylenealkyl has, without limitation, 5, 6, 10, 13, 18, 22, 27, 30, 33 or 39 carbon atoms.

In the present disclosure, the alicylyl is a carbocyclic group without a phenyl ring. Particularly, the alicylyl may comprise, but is not limited to, at least one of cyclopropyl, cyclobutyl, cyclopentyl, cyclohexyl, and cyclopentenyl. In an embodiment of the present disclosure, the substituted or unsubstituted alicylyl is a substituted or unsubstituted C₃-C₃₀ alicylyl. That is, the alicylyl has 3 to 30 carbon atoms. Particularly, the alicylyl has, without limitation, 3, 5, 9, 10, 13, 15, 18, 23, 26 or 30 carbon atoms.

In the present disclosure, the alicylidene is a divalent alicylyl. Particularly, the alicylidene may comprise, but is not limited to, at least one of cyclopropylene, cyclobutylene, cyclopentylene, cyclohexylene, and cyclopentenylene. In an embodiment of the present disclosure, the substituted or unsubstituted alicylidene is a substituted or unsubstituted C₃-C₃₀ alicylidene. That is, the alicylylene has 3 to 30 carbon atoms. Particularly, the alicylylene has, without limitation, 3, 5, 9, 10, 13, 15, 18, 23, 26 or 30 carbon atoms.

In the present disclosure, the substituted group (such as alkyl, alkylene, alkenyl, alkenylene, alkynyl, alkynylene, aryl, arylene, arylenealkyl, heteroaryl, heteroarylene, heteroarylenealkyl, and alicylidene) is a group substituted with a substituent. In an embodiment, the substituent comprises at least one of halo, nitrogen atom, oxygen atom, sulfur atom, hydroxyl, nitro, amino, mercapto, methoxy, and cyano.

In the present disclosure, R₁ and/or R₂ may be a single bond, that is, the amino group (-NH₂) can be directly reacted with the phenanthrolinyl group. In an embodiment, R₁ and R₂ are both a single bond, and the diamine compound has a chemical structural formula represented by Formula (I-1):

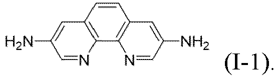

The diamine compound represented by Formula (I-1) has simple structure and much stable performance, to facilitate the preparation of a polyimide having stable performance.

In an embodiment of the present disclosure, R₁, and R₂ are independently selected from a single bond, substituted or unsubstituted C₆-C₃₀ arylene, or substituted or unsubstituted C₇-C₄₀ arylenealkyl. By means of this, the structural stability of the diamine compound is further improved. In an embodiment, R₁ and R₂ are the same group. In another embodiment, R₁ and R₂ are different groups. In an embodiment of the present disclosure, the diamine compound comprises one of the compounds represented by Formulas (I-1) to (I-4): 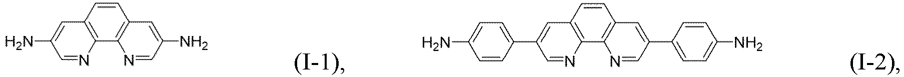 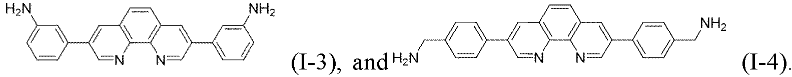 When R₁ and R₂ are both a single bond, the diamine compound has a chemical structural formula represented by Formula (I-1); when R₁ and R₂ are both an arylene, the diamine compound has a chemical structural formula represented by Formula (I-2) and Formula (I-3); and when R₁ and R₂ are both an arylenealkyl, the diamine compound has a chemical structural formula represented by Formula (I-4).

The present disclosure further provides a method for preparing a diamine compound, by which the diamine compound according to any one of the above embodiments can be prepared. The method comprises the following steps.

A first reactant is provided. The first reactant has a chemical structural formula represented by Formula (II), where R₃ and R₄ are independently selected from chlorine atom, bromine atom, iodine atom, or astatine atom,

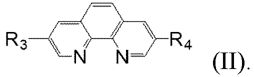

A second reactant is provided. The second reactant has a chemical structural formula represented by Formula (III), where R₅ is selected from hydrogen, an alkyl substituted with boric acid or a borate ester, an alkenyl substituted with boric acid or a borate ester, an alkynyl substituted with boric acid or a borate ester, an aryl substituted with boric acid or a borate ester, an aralkyl substituted with boric acid or a borate ester, a heteroaryl substituted with boric acid or a borate ester, a heteroarylalkyl substituted with boric acid or a borate ester, or an alicylyl substituted with boric acid or a borate ester,

H₂N-R₅ (III).

The first reactant and the second reactant are mixed under basic conditions to form a reaction solution, and reacted to obtain the diamine compound. The diamine compound has a chemical structural formula represented by Formula (I), where R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene,

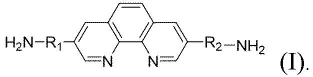

The method for preparing a diamine compound in the present disclosure is simple and convenient in operation, by which large-scale production of the diamine compound can be achieved, to promote the production and use of the polyimide.

In the present disclosure, R₃ and R₄ in the first reactant may be the same or different groups. In an embodiment, R₃ and R₄ are the same group, to facilitate the progress of the reaction. Particularly, the first reactant may be, but is not limited to, 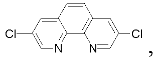 and 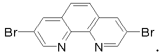 In the present disclosure, R₅ in the second reactant is selected from hydrogen, an alkyl substituted with boric acid or a borate ester, an alkenyl substituted with boric acid or a borate ester, an alkynyl substituted with boric acid or a borate ester, an aryl substituted with boric acid or a borate ester, an aralkyl substituted with boric acid or a borate ester, a heteroaryl substituted with boric acid or a borate ester, a heteroarylalkyl substituted with boric acid or a borate ester, or an alicylyl substituted with boric acid or a borate ester. Particularly, R₅ may be, but is not limited to, NH₃, 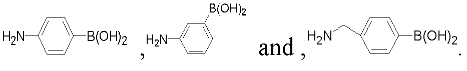 . In the present disclosure, the first reactant and the second reactant are subjected to a Suzuki coupling to prepare the diamine compound.

In an embodiment of the present disclosure, the reaction solution further comprises a catalyst and a catalyst ligand. The addition of the catalyst and the catalyst ligand promotes the progress of the reaction. In an embodiment of the present disclosure, the catalyst comprises a copper-based catalyst and a palladium-based catalyst. The copper-based catalyst comprises cuprous oxide, and the palladium (Pb)-based catalyst comprises at least one of bis(3,5,3',5'-dimethoxydibenzylideneacetone) palladium, bis(tri-tert-butyl) palladium, tris(dibenzylideneacetone) palladium, palladium chloride, palladium acetate, tetrakis(triphenylphosphine) palladium, and bis(tri-tert-butylphosphine) palladium. In an embodiment of the present disclosure, the catalyst ligand comprises at least of N,N'-dimethyl ethylenediamine, triphenylphosphine, 2-dicyclohexylphosphino-2',4',6'-triisopropylbiphenyl, 4,5-bis(diphenylphosphino)-9,9-dimethyl xanthene, triphenylphosphine oxide, and tris(o-methylphenyl) phosphine. The catalyst and catalyst ligand can effectively promote the progress of the Suzuki coupling. In an embodiment of the present disclosure, the molar ratio of the first reactant, the second reactant, the catalyst, and the catalyst ligand is 1: (2-50): (0.005-0.2): (0.005-0.5). This can facilitate the progress of the Suzuki coupling and improve the preparation efficiency of the diamine compound. In an embodiment, the molar ratio of the first reactant, the second reactant, the catalyst, and the catalyst ligand is 1: (3-45): (0.01-0.18): (0.01-0.45). In another embodiment, the molar ratio of the first reactant, the second reactant, the catalyst, and the catalyst ligand is 1: (8-37): (0.05-0.16): (0.1-0.4). In another embodiment, the molar ratio of the first reactant, the second reactant, the catalyst, and the catalyst ligand is 1: (10-25): (0.05-0.1): (0.1-0.3). In another embodiment, the molar ratio of the first reactant, the second reactant, the catalyst, and the catalyst ligand is 1: (30-50): (0.1-0.2): (0.3-0.5).

In an embodiment of the present disclosure, the reaction solution further comprises an alkaline substance, to facilitate the progress of the reaction under basic conditions. In an embodiment of the present disclosure, the alkaline substance comprises at least of potassium carbonate, sodium carbonate, cesium fluoride, sodium hydroxide, potassium hydroxide, and barium hydroxide. In an embodiment of the present disclosure, the molar ratio of the first reactant, the second reactant, and the alkaline substance is 1: (2-50): (0.05-10). This can facilitate the progress of the Suzuki coupling and improve the preparation efficiency of the diamine compound. In an embodiment, the molar ratio of the first reactant, the second reactant, and the alkaline substance is 1: (3-45): (0.1-9). In another embodiment, the molar ratio of the first reactant, the second reactant, and the alkaline substance is 1: (8-37): (2-8). In another embodiment, the molar ratio of the first reactant, the second reactant, and the alkaline substance is 1: (10-25): (1-4). In another embodiment, the molar ratio of the first reactant, the second reactant, and the alkaline substance is 1: (30-50): (5-8).

In an embodiment of the present disclosure, the reaction solution further comprises a solvent, and the solvent is used to dissolve and disperse the components in the reaction solution. In the present disclosure, the solvent may be, but is not limited to, water, ethylene glycol, and 1,4-dioxane. In an embodiment, the solvent may be a mixture of water and 1,4- dioxane. Particularly, the volume ratio of 1,4-dioxane to water may be, but is not limited to, 2 to 10.

In an embodiment of the present disclosure, the reaction temperature is 25°C to 180°C, and the reaction time is 2 h to 72 h. This can ensure the progress of the reaction, improve the preparation efficiency of the diamine compound, and avoid the occurrence of side reactions. Particularly, the reaction temperature in the preparation of the diamine compound may be, but is not limited to, 25°C, 30°C, 40°C, 45°C, 60°C, 80°C, 100°C, 115°C, 130°C, 145°C, or 180°C, and the reaction time may be, but is not limited to, 2 h, 8 h, 10 h, 20 h, 35 h, 40 h, 55 h, 65 h, or 70 h. In an embodiment, the reaction temperature is 45°C to 90°C, and the reaction time is 35 h to 72 h. In another embodiment, the reaction temperature is 100°C to 180°C, and the reaction time is 2 h to 30 h. In an embodiment of the present disclosure, the reaction can be carried out under an inert gas atmosphere, to avoid the occurrence of side reactions. Particularly, the inert gas may be, but is not limited to, argon, nitrogen and the like. Particularly, after the reaction, the diamine compound can be obtained by separation and purification.

In an embodiment of the present disclosure, the first reactant is reacted with NH₃ to prepare the diamine compound represented by Formula (I-1). In an embodiment, 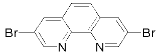 is mixed with aqueous ammonia, and reacted to prepare the compound represented by Formula (I-1). Particularly, a catalyst, a catalyst ligand, an alkaline substance, and the like can also be added. In a specific embodiment, under a nitrogen atmosphere, brominated 1,10-phenanthroline, 28% concentrated aqueous ammonia, a copper-based catalyst (cuprous oxide), N,N'-dimethylethylenediamine, and potassium carbonate are dissolved in ethylene glycol, and stirred at 45°C to 180°C for 2 h to 72 h, to obtain the diamine compound of Formula (I-1). Particularly, the molar ratio of the brominated 1,10-phenanthroline, the 28% concentrated aqueous ammonia, the copper-based catalyst (cuprous oxide), N,N'-dimethylethylenediamine, and potassium carbonate may be, but is not limited to, 1: (10-50): (0.01-0.1): (0.05-0.5): (0.05-0.5). The reaction scheme of the above reaction is shown below:

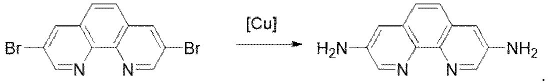

In an embodiment of the present disclosure, 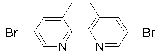 is mixed with 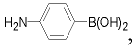 and reacted to prepare the compound represented by Formula (I-2). Particularly, a catalyst, a catalyst ligand, an alkaline substance, and the like can also be added. In a specific embodiment, under a nitrogen atmosphere, 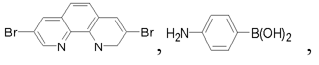 , a palladium-based catalyst, a catalyst ligand, and an alkaline substance are dissolved in a solvent, and stirred at 45°C to 120°C for 2 h to 72 h, to obtain the diamine compound of Formula (I-2). Particularly, the molar ratio of 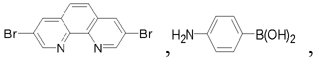 , the palladium-based catalyst, the catalyst ligand, and the alkaline substance may be, but is not limited to, 1: (2-4): (0.01-0.1): (0.01-0.5): (2-10). The reaction scheme of the above reaction is shown below:

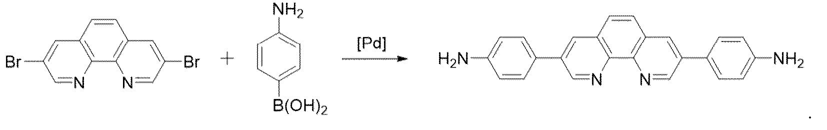

In an embodiment of the present disclosure, 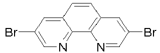 is mixed with 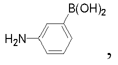 and reacted to prepare the compound represented by Formula (I-3). Particularly, a catalyst, a catalyst ligand, an alkaline substance, and the like can also be added. In a specific embodiment, under a nitrogen atmosphere, 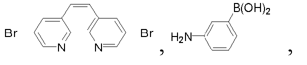 , a palladium-based catalyst, a catalyst ligand, and an alkaline substance are dissolved in a solvent, and stirred at 45°C to 120°C for 2 h to 72 h, to obtain the diamine compound of Formula (I-3). Particularly, the molar ratio of 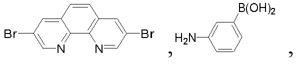 , the palladium-based catalyst, the catalyst ligand, and the alkaline substance may be, but is not limited to, 1: (2-4): (0.01-0.1): (0.01-0.5): (2 to 10). The reaction scheme of the above reaction is shown below:

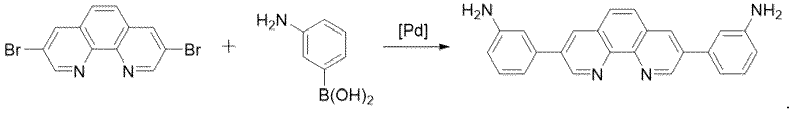

In an embodiment of the present disclosure, 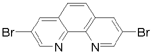 is mixed with 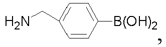 and reacted to prepare the compound represented by Formula (I-4). Particularly, a catalyst, a catalyst ligand, an alkaline substance, and the like can also be added. In a specific embodiment, under a nitrogen atmosphere, 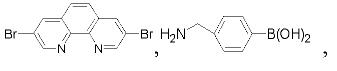 , a palladium-based catalyst, a catalyst ligand, and an alkaline substance are dissolved in a solvent, and stirred at 45°C to 120°C for 2 h to 72 h, to obtain the diamine compound of Formula (I-4). Particularly, the molar ratio of 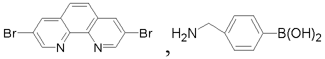 , the palladium-based catalyst, the catalyst ligand, and the alkaline substance may be, but is not limited to, 1: (2-4): (0.01-0.1): (0.01-0.5): (2-10). The reaction scheme of the above reaction is shown below:

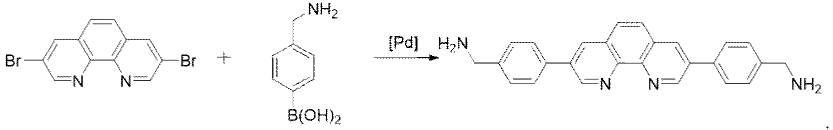

The present disclosure provides a method for preparing a polyimide acid, which comprises the following steps. A first diamine compound according to any of the above embodiments is mixed with a dianhydride to form a mixed solution, and reacted to obtain the polyimide acid. The polyimide acid comprises a repeat unit represented by Formula (IV), where R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R₇ is selected from a substituted or unsubstituted arylene or substituted or unsubstituted heteroarylene,

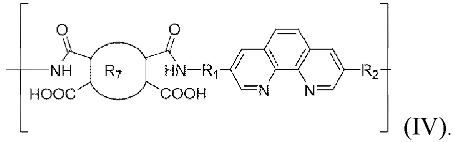

The method for preparing a polyimide acid provided in the present disclosure is simple and convenient in operation, by which large-scale production of the polyimide acid can be achieved. The prepared polyimide acid has a 1,10-phenanthrolinyl group, which can form a chemical bond with a metal member, to improve the adhesion to the metal member, thus promoting the preparation of the polyimide and improving the bonding strength between the polyimide and the metal member. It can be understood that R₁ and R₂ in the diamine compound represented by Formula (I) and the repeat unit in the polyimide acid represented by Formula (IV) are the same.

In the present disclosure, R₇ is selected from a substituted or unsubstituted arylene or substituted or unsubstituted heteroarylene, and the R₇ group shares two carbon atoms with a group on either side of the R₇ group. For example, when R₇ is phenyl, the repeat unit represented by Formula (IV) is 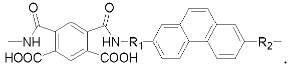 In an embodiment of the present disclosure, the number of repeat unit represented by Formula (IV) in the polyimide acid may be, but is not limited to, 5 to 2000. In an embodiment, the number of repeat unit represented by Formula (IV) in the polyimide acid may be 20 to 2000. In another embodiment, the number of repeat unit represented by Formula (IV) in the polyimide acid may be 100 to 1800. In another embodiment, the number of repeat unit represented by Formula (IV) in the polyimide acid may be 300 to 1500.

In an embodiment of the present disclosure, the dianhydride comprises at least of pyromellitic dianhydride, 2,3,3',4'-diphenyl ether tetracarboxylic dianhydride, 3,3',4,4'-benzophenone tetracarboxylic dianhydride, 3,3',4,4'-diphenyl sulfone tetracarboxylic dianhydride, 2,2-bis(3,4-dicarboxyphenyl)hexafluoropropane dianhydride, 4,4'-bis(3,4-dicarboxyphenoxy)diphenyl ether dianhydride, 4,4'-bis(3,4-dicarboxyphenoxy)benzophenone dianhydride, 4,4'-bis(3,4-dicarboxyphenoxy)diphenylmethane dianhydride, and 4,4'-bis(3,4-dicarboxyphenoxy)diphenyl sulfone dianhydride. The choice of the dianhydride determines the R₇ group.

In an embodiment of the present disclosure, the molar ratio of the first diamine compound to the dianhydride is 0.9 to 1.1. This promotes the rapid preparation of the polyimide acid. Particularly, the molar ratio of the first diamine compound to the dianhydride may be, but is not limited to, 0.9, 0.95, 1, 1.05, or 1.1.

In an embodiment of the present disclosure, the mixed solution further comprises a solvent. The components in the mixed solution are mixed and dispersed in the solvent. In an embodiment of the present disclosure, the solvent may comprise at least one of dimethyl sulfoxide, N-methylpyrrolidone, N,N-dimethylformamide, N,N-dimethylacetamide, and m-cresol.

In an embodiment of the present disclosure, the reaction temperature is 0°C to 100°C, and the reaction time is 2h to 12h. Particularly, the reaction temperature in the preparation of the polyimide acid may be, but is not limited to, 0°C, 5°C, 10°C, 15°C, 20°C, 25°C, 30°C, 40°C, 45°C, 50°C, 60°C, 70°C, 80°C, 90°C, or 100°C, and the reaction time may be, but is not limited to, 2 h, 3 h, 5 h, 6 h, 8 h, 9 h, or 12 h. In an embodiment, the reaction temperature is 45°C to 60°C, and the reaction time is 7 h to 12 h. In another embodiment, the reaction temperature is 60°C to 100°C, and the reaction time is 2 h to 7 h. In another embodiment, the reaction temperature is 0°C to 50°C, and the reaction time is 3 h to 7 h.

In an embodiment of the present disclosure, the mixed solution further comprises a second diamine compound. By adding the second diamine compound, the film-forming performance of the polyimide acid and polyimide is improved. In an embodiment of the present disclosure, the second diamine compound comprises at least one of 4,4'-diaminodiphenyl ether, m-phenylenediamine, p-phenylenediamine, 4,4'-diaminodiphenyl sulfone, 1,3-diamino-2-methylpropane, N,N-bis(4-aminophenyl)-1,4-phenylenediamine, 9,9-bis(4-aminophenyl)fluorene, 1,2-diaminocyclohexane, and ethylenediamine. The second diamine compound has rotatability, such as sp3 hybridized oxygen, to improve the film forming performance of the polyimide acid. In an embodiment, the content of the second diamine compound in the mixed solution is less than that of the first diamine compound. In a specific embodiment, the mixed solution comprises a second diamine compound, which can ensure the film-forming performance of the polyimide film in the subsequent preparation of the polyimide film.

In an embodiment of the present disclosure, when the mixed solution contains the second diamine compound, the polyimide acid has a chemical structural formula represented by Formula (V): 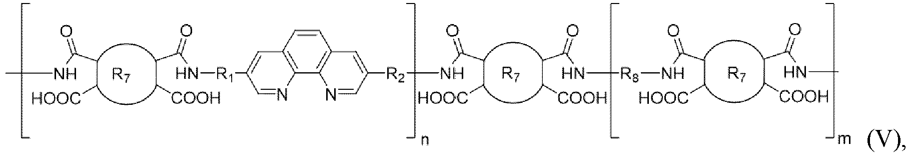 where R₈ is selected from a substituted or unsubstituted alkylene, substituted or unsubstituted arylene, or substituted or unsubstituted heteroarylene. The choice of the second diamine compound determines the R₈ group.

In an embodiment of the present disclosure, n may be 5 to 2000. In an embodiment, n is 20 to 2000. In another embodiment, n is 20 to 2000. In another embodiment, n is 100 to 1800. In another embodiment, n is 300 to 1500. In another embodiment, n is 500 to 1000. In another embodiment, n is 1000 to 1500.

In an embodiment of the present disclosure, m is less than or equal to 1000. In an embodiment, m is 0 to 1000. That is, the mixed solution contains no the second diamine compound, m is 0. In another embodiment, m is 10 to 900. In another embodiment, m is 100 to 850. In another embodiment, m is 200 to 700. In another embodiment, m is 200 to 500. In another embodiment, m is 500 to 800.

The present disclosure provides a polyimide acid. The polyimide acid comprises a repeat unit represented by Formula (IV): 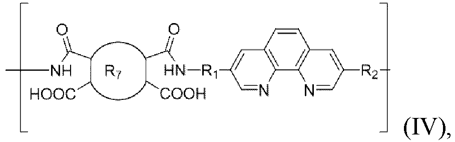 where R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R₇ is selected from a substituted or unsubstituted arylene or substituted or unsubstituted heteroarylene. The polyimide acid provided in the present disclosure has a 1,10-phenanthrolinyl group, which can form a chemical bond with a metal member, to improve the adhesion to the metal member, thus promoting the preparation of the polyimide and improving the bonding strength between the polyimide and the metal member. In the present disclosure, the polyimide acid can be prepared according to any embodiment of the preparation method of the polyimide acid.

The present disclosure provides a method for preparing a polyimide. The method comprises the following steps. The polyimide acid according to any one of the above embodiments is imidized to obtain the polyimide. The polyimide comprises a repeat unit represented by Formula (VI), where R₁, R₂, and R₇ are the same as those in the polyimide acid,

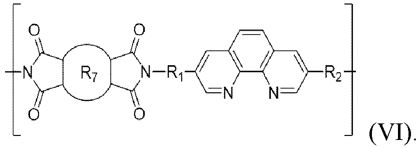

The method for preparing a polyimide provided in the present disclosure is simple and convenient in operation, by which large-scale production of the polyimide can be achieved. The prepared polyimide has a 1,10-phenanthrolinyl group, which can form a chemical bond with a metal member, to improve the adhesion of the polyimide to the metal member, thus promoting the use of the polyimide.

In the present disclosure, the imidization may be, but is not limited to, heat treatment. The polyimide acid is converted into the polyimide after heat treatment. In an embodiment of the present disclosure, when the second diamine compound is used in the preparation process of the polyimide acid, the polyimide has a chemical structural formula represented by Formula (VII), where R₈ is the same as that in the polyimide acid,

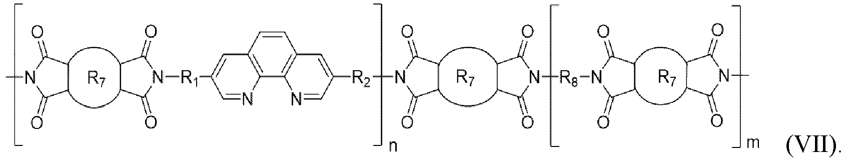

When the second diamine compound is not used in the preparation process of the polyimide acid, m is 0 in the chemical structure of the polyimide represented by Formula (VII).

The present disclosure provides a polyimide. The polyimide comprises a repeat unit represented by Formula (VI): 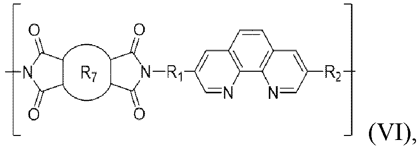 where R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R₇ is selected from a substituted or unsubstituted arylene or substituted or unsubstituted heteroarylene. The polyimide provided in the present disclosure has a 1,10-phenanthrolinyl group, which can form a chemical bond with a metal member, to improve the adhesion of the polyimide to the metal member, thus promoting the use of the polyimide. In the present disclosure, the polyimide can be prepared according to any embodiment of the preparation method of the polyimide. The polyimide provided in the present disclosure has good thermal stability, and can still retain a rigid structure at a high temperature, which is beneficial to its use. In an embodiment, the polyimide can still retain a good stability at 500°C or higher.

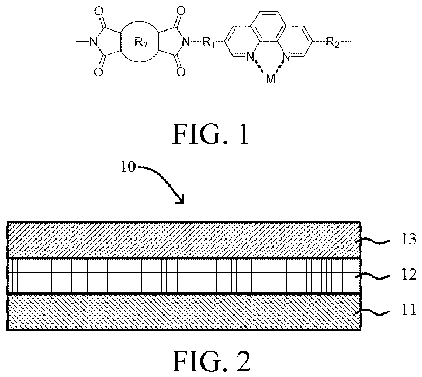

FIG. 1, schematically shows the bonding of a polyimide to a metal atom provided in an embodiment of the present disclosure, where m is a metal element, and the dotted line is a coordination bond formed between the polyimide and the metal. The polyimide provided in the present disclosure can form, together with the metal element, a coordination compound having a five-membered ring, whereby the bonding strength between the polyimide film and the metal surface can be increased when the polyimide is used. Particularly, the metal element may be, but is not limited to, aluminum, copper, iron, zinc, titanium, and the like.

The present disclosure further provides a method for preparing a polyimide film, which comprises the following steps. The polyimide acid is coated and then imidized to obtain the polyimide film. In an embodiment, the imidization comprises heat treatment at 80°C to 400°C for 1 h to 10 h. Particularly, the imidization temperature may be, but is not limited to, 120°C, 150°C, 180°C, 200°C, 230°C, 250°C, 290°C, 310°C, or 370°C. The imidization time may be, but is not limited to, 1 h, 3 h, 7 h, 9 h, or 10 h. In another embodiment, the imidization comprises heat treatment at 80°C to 200°C for 1 h to 6 h, followed by heating to 200°C to 400°C and heat treatment for 1 h to 4 h. By heat treatment at an elevated temperature, the imidization efficiency can be improved. Further, the heating rate may be 1°C/min to 7°C/min. Particularly, the heating rate may be, but is not limited to, 2°C/min, 3°C/min, 4°C/min, 5°C/min, or 6°C/min. In a specific embodiment, the imidization comprises heat treatment at 120°C for 1 h, followed by heating to 250°C and heat treatment for 1 h, and heating to 350°C and heat treatment for 1 h, where the heating rate is 2°C/min. In another specific embodiment, the imidization comprises heat treatment at 80°C for 2 h, followed by heating to 120°C and heat treatment for 1 h, heating to 160°C and heat treatment for 1 h, heating to 180°C and heat treatment for 1 h, heating to 240°C and heat treatment for 1 h, heating to 280°C and heat treatment for 1 h, and heating to 350°C and heat treatment for 1 h, where the heating rate is 2°C/min.

In the present disclosure, the present inventor finds through research that when R₁, R₂ in Formula (I) are independently selected from a single bond, substituted or unsubstituted arylene, substituted or unsubstituted heteroarylene, or substituted or unsubstituted alicylidene, the film forming performance of the polyimide acid and polyimide will be reduced if the second diamine compound is not added during the preparation of the polyimide acid and polyimide. Therefore, when R₁ and R₂ are independently selected from a single bond, substituted or unsubstituted arylene, substituted or unsubstituted heteroarylene, or substituted or unsubstituted alicylidene, the film forming performance of the polyimide acid and polyimide can be increased by adding the second diamine compound. In an embodiment, when the polyimide acid or polyimide is prepared with one of the compounds represented by Formulas (I-1) to (I-4), the second diamine compound can be added, to improve the film forming performance of the polyimide acid or polyimide.

The present disclosure provides a battery assembly, which comprises a polyimide film, wherein the polyimide film is made of a polyimide according to any one of the above embodiments. FIG. 2, is a schematic cross-sectional view of a battery assembly provided in an embodiment of the present disclosure. A battery assembly 10 comprises a heat dissipation component 13, a polyimide film 12 arranged on a surface of the heat dissipation component 13, and a battery core 11 arranged on a surface of the polyimide film 12. In related art, because the battery core 11 will generate heat during use, the heat dissipation component 13 needs to be arranged to transfer the heat generated by the battery core 11, thereby reduce the temperature of the battery core 11 and ensuring the life and safety of the battery core 11 during use. When the heat dissipation component 13 is arranged on the surface of the battery core 11, the heat dissipation component 13 and the battery core 11 need to be connected, to ensure the bonding strength therebetween, and to ensure that no relative movement occurs during use. When a connecting structure is used, the weight of the battery assembly 10 is unduly increased, and the connecting structure may also scratch the surface of the heat dissipation component 13, thus reducing the safety during use. In the battery assembly 10 provided in the present disclosure, the battery core 11 and the heat dissipation component 13 are connected by the polyimide film 12. Since the polyimide used in the polyimide film 12 has a 1,10-phenanthrolinyl group, the bonding strength between the polyimide film 12 and the heat dissipation component 13 is increased, such that the polyimide film 12 used for insulation will not fall off during use, thereby improving the safety and life of the battery assembly 10. Moreover, in related art, with the increase of the energy density of the battery core 11, insulation treatment is needed between the battery core 11 and the heat dissipation component 13, to ensure the safety of the battery assembly 10. With the application and development of the cell-to-body (CTB) technology of the battery core 11, the battery assembly 10 is located below the vehicle seat and needs to bear the weight of passengers, raising a higher requirement for the safety of the battery assembly 10. The polyimide film 12 made of the polyimide provided in the present disclosure is insulating, which further improves the safety of the battery assembly 10 during use.

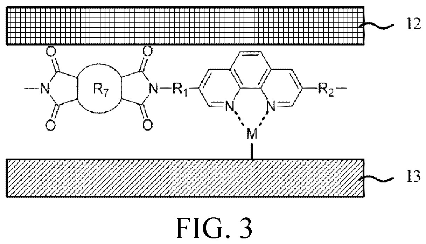

In an embodiment of the present disclosure, the surface of the heat dissipation component 13 is of a metal material. This facilitates the formation of a chemical bond between the polyimide film 12 and the surface of the heat dissipation component 13, and improves the bonding strength. Particularly, the metal material may be, but is not limited to, at least one of aluminum, copper, an aluminum alloy, and a copper alloy, or stainless steel. In the present disclosure, the heat dissipation component 13 may be, but is not limited to, a liquid-cooled plate or the like. FIG. 3, schematically shows the bonding of the polyimide film 12 to the heat dissipation component 13 provided in an embodiment of the present disclosure. in which the surface of the heat dissipation component 13 is of a metal material, M is a metal element, and the dotted line is a coordination bond formed between the polyimide in the polyimide film 12 and the metal element in the heat dissipation component 13. In an embodiment of the present disclosure, the surface of the battery core 11 is of a metal material. A chemical bond is formed between the polyimide film 12 and the surface of the battery core 11, which improves the bonding strength. Particularly, the metal material may be, but is not limited to, at least one of aluminum, copper, an aluminum alloy, and a copper alloy, or stainless steel.

The present disclosure provides an electronic device, which comprises a battery assembly 10 according to any one of the above embodiments. The electronic device may be, but is not limited to, a vehicle, a cell phone, a server, and a computer, etc. The battery assembly 10 in the electronic device provided in the present disclosure has a high safety, thus promoting the use of the electronic device.

The technical solution of the present disclosure will be further explained by specific examples and comparative examples.

### Example 1

A diamine compound is prepared through a method as follows.

Under a nitrogen atmosphere, cuprous oxide (catalyst), brominated 1,10-phenanthroline, 28% concentrated aqueous ammonia, potassium carbonate (alkaline substance), and N,N'-dimethylethylenediamine (catalyst ligand) (molar ratio 0.1:1:50:0.5:0.5) are dissolved in ethylene glycol, and stirred at 180°C for 72 h. After reaction, the solution is cooled to room temperature, extracted with ethyl acetate, and purified by column chromatography to obtain the diamine compound of Formula (I-1).

### Example 2

A diamine compound is prepared through a method as follows.

Under a nitrogen atmosphere, palladium acetate (catalyst), triphenylphosphine (catalyst ligand), brominated 1,10-phenanthroline, aminophenylboronic acid hydrochloride, and sodium hydroxide (alkaline substance) (molar ratio 0.02:0.05:1:2.1:8) are dissolved in 1,4-dioxane, and stirred at 100°C for 18 h. After reaction, the solution is cooled to room temperature, filtered, and purified by column chromatography to obtain the diamine compound of Formula (I-2).

### Example 3

A diamine compound is prepared through a method as follows.

Under a nitrogen atmosphere, bis(3,5,3',5'-dimethoxydibenzylideneacetone) palladium (catalyst), triphenylphosphine (catalyst ligand), brominated 1,10-phenanthroline, aminophenylboronic acid hydrochloride, and potassium carbonate (alkaline substance)( molar ratio 0.01:0.02:1:2.1:8) are dissolved in 1,4-dioxane and water (volume ratio 5:1), and stirred at 100°C for 18 h. After reaction, the solution is cooled to room temperature, filtered, and purified by column chromatography to obtain the diamine compound of Formula (I-3).

### Example 4

A diamine compound is prepared through a method as follows.

Under a nitrogen atmosphere, palladium acetate (catalyst), triphenylphosphine (catalyst ligand), brominated 1,10-phenanthroline, 4-(aminomethyl)phenyl)boric acid, and potassium carbonate (alkaline substance) (molar ratio 0.01:0.02:1:2.1:8) are dissolved in 1,4-dioxane and stirred at 100°C for 12 h. After reaction, the solution is cooled to room temperature, filtered, and purified by column chromatography to obtain the diamine compound of Formula (I-4).

### Example 5

A polyimide acid is prepared through a method as follows.

10.5 g of the diamine compound prepared in Example 1, 10 g of 4,4'-diaminodiphenyl ether, 160 g of N,N-dimethylformamide are sequentially added to a reactor, stirred, and adjusted to a temperature of 60°C. 21.8 g of pyromellitic anhydride is slowly added into the reactor, stirred for 6 h, and then cooled to room temperature, to obtain a polyimide acid.

### Example 6

A polyimide acid is prepared through a method as follows.

18.1g of the diamine compound prepared in Example 2, 10 g of 4,4'-diaminodiphenyl ether, 180 g of N,N-dimethylformamide are sequentially added to a reactor, stirred, and adjusted to a temperature of 60°C. 21.8 g of pyromellitic anhydride is slowly added into the reactor, stirred for 12 h, and then cooled to room temperature, to obtain a polyimide acid.

### Example 7

A polyimide acid is prepared through a method as follows.

18.1g of the diamine compound prepared in Example 3, 10 g of 4,4'-diaminodiphenyl ether, 160 g of N,N-dimethylformamide are sequentially added to a reactor, stirred, and adjusted to a temperature of 45°C. 21.8 g of pyromellitic anhydride is slowly added into the reactor, stirred for 4 h, and then cooled to room temperature, to obtain a polyimide acid.

### Example 8

A polyimide acid is prepared through a method as follows.

39g of the diamine compound prepared in Example 4, 160 g of N,N-dimethylformamide are sequentially added to a reactor, stirred, and adjusted to a temperature of 0°C. 21.8 g of pyromellitic anhydride is slowly added into the reactor, stirred for 6 h, and then cooled to room temperature, to obtain a polyimide acid.

### Comparative Example 1

A polyimide acid is prepared through a method as follows.

20 g of 4,4'-diaminodiphenyl ether and 160 g of N,N-dimethylformamide are sequentially added to a reactor, stirred, and adjusted to a temperature of 45 °C. 21.8 g of pyromellitic anhydride is slowly added into the reactor, stirred for 4 h, and then cooled to room temperature, to obtain a polyimide acid.

### Performance test

The structure of the diamine compounds obtained in Examples 1-4 is characterized by nuclear magnetic resonance. The results of 1H NMR are as follows:

Formula (I-1): 1H NMR (500 MHz, Chloroform-d) δ 8.44 (d, 1H), 7.49 (m, 2H), 4.51 (d, 1H), 4.40 (d, 1H);

Formula (I-2): 1H NMR (500 MHz, Chloroform-d) δ 8.88 (d, 2H), 8.38 (m, 2H), 7.87 (t, 2H), 7.63 -7.57 (m, 2H), 6.67-6.1 (m, 2H), 4.20 (d, 1H), 4.13 (d, 1H);

Formula (I-3): 1H NMR (500 MHz, Chloroform-d) δ 8.89 (d, 2H), 8.46 (m,21H), 7.87 (t, 2H), 7.29 (m, 2H), 7.28 (m, 2H), 6.98 (t, 2H), 6.68-6.62 (m, 2H), 4.38 (d, 2H), 4.29 (d, 2H); and

Formula (I-4): 1H NMR (500 MHz, Chloroform-d) δ 8.95 (d, J = 2.0 Hz, 1H), 8.61 (q, J = 1.4 Hz, 1H), 7.87 (t, J = 1.3 Hz, 1H), 7.68 - 7.63 (m, 2H), 7.30 (dt, J = 8.0, 1.1 Hz, 2H), 4.05 (tt, J = 6.2, 1.0 Hz, 2H), 2.52 (dt, J = 7.1, 6.2 Hz, 1H), 2.36 (dt, J = 7.0, 6.2 Hz, 1H).

The polyimide acids prepared in Examples 5-8 and Comparative Example 1 are respectively coated and cast on the surface of an aluminum plate to prepare a wet film of a corresponding solution. The wet film is transferred to an oven, and imidization is carried out heating according to the following heating procedure: 80°C/2h, 120°C/1h, 160°C/1h, 180°C/1h, 240°C/1h, 280°C/1h, and 350°C/1h, where the heating rate is 2°C/min. A polyimide film formed on the surface of the aluminum plate is obtained. According to ASTM D3359 Method B Cross-cut tape test, the bonding strength between the polyimide film and the aluminum plate is tested, and the bonding strength is obtained, as shown in Table 1. Moreover, the decomposition temperature 5% weight loss of the formed polyimide film is detected according to GB/T 13464-2008. The results are shown in Table 1. The polyimide acids prepared in Examples 5 to 8 and Comparative Example 1 are respectively coated and cast on the surface of a glass plate to prepare a wet film of a corresponding solution. The wet film is transferred to an oven, and imidization is carried out following the same heating procedure as above. A polyimide film formed on the surface of the glass plate is obtained. After the glass plate is cooled to room temperature, it is soaked in deionized water, and the polyimide film is peeled off, to obtain a corresponding self-supporting polyimide film. The film forming performance is evaluated specifically as follows. The film forming performance is considered very good if the film is formed into a whole piece of film material on the glass, and the film surface is not broken and can remain intact after peeling. The film forming performance is considered normal if the film is formed into a whole piece of film material on the glass, and the film surface is not broken, but remains non-intact after peeling. The film forming performance is considered poor if the film is broken into pieces on the glass. It can be seen from Table 1 that compared with Comparative Example 1, the decomposition temperature at 5% weight loss of the polyimide film provided in the present disclosure is higher indicating a very excellent heat stability. Compared with Example 7, the amino group in the diamine compound used and the polyimide prepared in Example 6 is on the rotation axis of the phenyl ring, which enhances the linearity and further improves the heat stability. The 1,10-phenanthrolinyl group in the polyimide film can form a coordination bond with aluminum, so that the bonding strength between the polyimide film and the aluminum plate is high, thus promoting the use of the polyimide.

**Table 1. Performance test results**

| | Bonding strength | Decomposition temperature at 5% weight loss | Film forming performance |
|---|---|---|---|
| Example 5 | 5B | 540°C | Very good |
| Example 6 | 5B | 550°C | Very good |
| Example 7 | 5B | 500°C | Very good |
| Example 8 | 5B | 515°C | Very good |
| Comparative Example 1 | 0B | 480°C | Very good |

The diamine compound, the polyimide acid and the polyimide provided in the present disclosure have a 1,10-phenanthrolinyl group, which can form a chemical bond with a metal member, to improve the bonding strength with the metal member, thus promoting the use in electronic devices.

The above embodiments are only several implementations of the present application, and are described in detail, which, however, are not to be construed as a limitation to the scope of the present disclosure. It is to be understood that for a person of ordinary skill in the art, several variations and improvements can be made by those of ordinary skill in the art without departing from the idea of the present disclosure, which are all contemplated in the protection scope of the present disclosure. Therefore, the protection scope of the present disclosure shall be defined by the appended claims.

## Claims

1. A diamine compound, having a chemical structural formula represented by Formula (I), 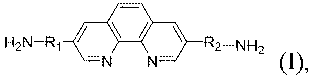 wherein R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene.

2. The diamine compound according to claim 1, wherein the substituted or unsubstituted alkylene is a substituted or unsubstituted C₁-C₈ alkylene;
the substituted or unsubstituted alkenylene is a substituted or unsubstituted C₂-C₈ alkenylene;
the substituted or unsubstituted alkynylene is a substituted or unsubstituted C₂-C₈ alkynylene;
the substituted or unsubstituted arylene is a substituted or unsubstituted C₆-C₃₀ arylene;
the substituted or unsubstituted arylene is a substituted or unsubstituted C₇-C₄₀ arylenealkyl;
the substituted or unsubstituted heteroarylene is a substituted or unsubstituted C₂-C₃₀ heteroarylene;
the substituted or unsubstituted arylene is a substituted or unsubstituted C₃-C₄₀ heteroarylenealkyl; and
the substituted or unsubstituted alicylidene is a substituted or unsubstituted C₃-C₃₀ alicylidene.

3. The diamine compound according to claim 1, wherein R₁ and R₂ are independently selected from a single bond, substituted or unsubstituted C₆-C₃₀ arylene, or substituted or unsubstituted C₇-C₄₀ arylenealkyl.

4. The diamine compound according to claim 3, wherein the diamine compound comprises one of the compounds represented by Formulas (I-1) to (I-4):

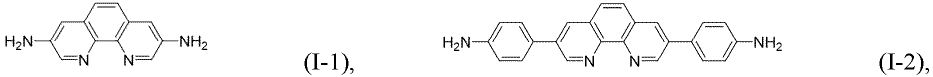

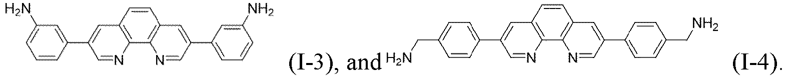

5. A method for preparing a diamine compound, comprising:
providing a first reactant, having a chemical structural formula represented by Formula (II), wherein R₃ and R₄ are independently selected from chlorine atom, bromine atom, iodine atom, or astatine atom, 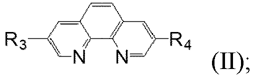
providing a second reactant, having a chemical structural formula represented by Formula (III), wherein R₅ is selected from hydrogen, an alkyl substituted with boric acid or a borate ester, an alkenyl substituted with boric acid or a borate ester, an alkynyl substituted with boric acid or a borate ester, an aryl substituted with boric acid or a borate ester, an aralkyl substituted with boric acid or a borate ester, a heteroaryl substituted with boric acid or a borate ester, a heteroarylalkyl substituted with boric acid or a borate ester, or an alicylyl substituted with boric acid or a borate ester,
H₂N-R₅ (III);
and
mixing the first reactant and the second reactant under basic conditions to form a reaction solution, and reacting to obtain the diamine compound, having a chemical structural formula represented by Formula (I), wherein R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene,

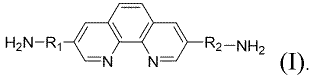

6. **The preparation** method according to claim 5, wherein the reaction solution further comprises a catalyst and a catalyst ligand;
the catalyst comprises a copper-based catalyst and a palladium-based catalyst, wherein the copper-based catalyst comprises cuprous oxide, and the palladium-based catalyst comprises at least one of bis(3,5,3',5'-dimethoxydibenzylideneacetone) palladium, bis(tri-tert-butyl) palladium, tris(dibenzylideneacetone) palladium, palladium chloride, palladium acetate, tetrakis(triphenylphosphine) palladium, and bis(tri-tert-butylphosphine) palladium;
the catalyst ligand comprises at least one of N,N'-dimethyl ethylenediamine, triphenylphosphine, 2-dicyclohexylphosphino-2',4',6'- triisopropylbiphenyl, 4,5-bis(diphenylphosphino)-9,9-dimethyl xanthene, triphenylphosphine oxide, and tris(o-methylphenyl) phosphine;
the molar ratio of the first reactant, the second reactant, the catalyst, and the catalyst ligand is 1: (2-50): (0.005-0.2): (0.005-0.5);
the reaction solution further comprises an alkaline substance, and the alkaline substance comprises at least one of potassium carbonate, sodium carbonate, cesium fluoride, sodium hydroxide, potassium hydroxide, and barium hydroxide;
the molar ratio of the first reactant, the second reactant, and the alkaline substance is 1: (2-50): (0.05-10); and
the reaction temperature is 25°C to 180°C, and the time is 2 h to 72 h.

7. The preparation method according to claim 5 or 6, wherein the reaction solution further comprises a solvent, and the solvent comprises water, ethylene glycol, and 1,4-dioxane, wherein the volume ratio of 1,4- dioxane to water is 2 to 10.

8. The preparation method according to any one of claims 5 to 7, wherein the reaction is carried out under an inert atmosphere.

9. A polyimide acid, comprising a repeat unit represented by Formula (IV), 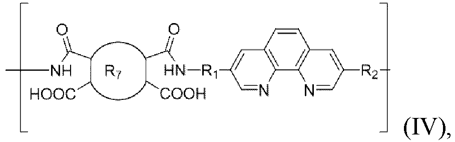 wherein R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R₇ is selected from a substituted or unsubstituted arylene or substituted or unsubstituted heteroarylene.

10. The polyimide acid according to claim 9, wherein the polyimide acid has a chemical structural formula represented by Formula (V), 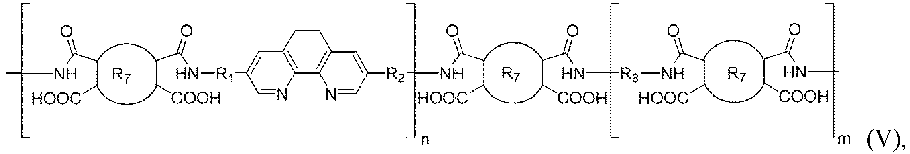 wherein R₈ is selected from substituted or unsubstituted alkylene, substituted or unsubstituted arylene, or substituted or unsubstituted heteroarylene, n is 5 to 2000, and m is 0 to 1000.

11. A method for preparing a polyimide acid, comprising mixing a first diamine compound with a dianhydride to form a mixed solution, and reacting to obtain the polyimide acid, wherein the first diamine compound is a diamine compound according to any one of claims 1 to 4 or a diamine compound prepared through the preparation method according to any one of claims 5 and 6, and comprise a repeat unit represented by Formula (IV), wherein R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R₇ is selected from a substituted or unsubstituted arylene or substituted or unsubstituted heteroarylene,

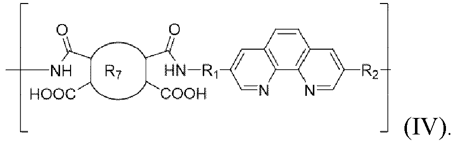

12. The preparation method according to claim 11, wherein the molar ratio of the first diamine compound to the dianhydride is 0.9 to 1.1;
the dianhydride comprises at least one of pyromellitic dianhydride, 2,3,3',4'-diphenyl ether tetracarboxylic dianhydride, 3,3',4,4'-benzophenone tetracarboxylic dianhydride, 3,3',4,4'-diphenyl sulfone tetracarboxylic dianhydride, 2,2-bis(3,4-dicarboxyphenyl)hexafluoropropane dianhydride, 4,4'-bis(3,4-dicarboxyphenoxy)diphenyl ether dianhydride, 4,4'-bis(3,4-dicarboxyphenoxy)benzophenone dianhydride , 4,4'-bis(3,4-dicarboxyphenoxy)diphenylmethane dianhydride, and 4,4'-bis(3,4-dicarboxyphenoxy)diphenyl sulfone dianhydride;
the mixed solution further comprises a second diamine compound, and the second diamine compound comprises at least one of 4,4'-diaminodiphenyl ether, m-phenylenediamine, p-phenylenediamine, 4,4'-diaminodiphenyl sulfone, 1,3-diamino-2-methylpropane, N,N-bis(4-aminophenyl)-1,4-phenylenediamine, 9,9-bis(4-aminophenyl)fluorene, 1,2-diaminocyclohexane, and ethylenediamine;
the mixed solution further comprises a solvent, and the solvent comprises at least one of dimethyl sulfoxide, N-methylpyrrolidone, N,N-dimethylformamide, N,N-dimethylacetamide, and m-cresol; and
the reaction temperature is 0°C to 100°C, and the time is 2 h to 12 h.

13. A polyimide, comprising a repeat unit represented by Formula (VI), 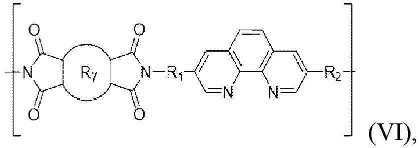 wherein R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R₇ is selected from a substituted or unsubstituted arylene or substituted or unsubstituted heteroarylene.

14. The polyimide according to claim 13, wherein the polyimide has a chemical structural formula represented by Formula (VII), 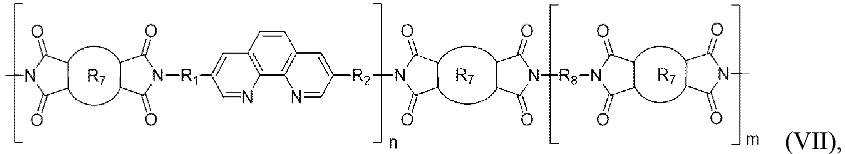 wherein R₈ is selected from substituted or unsubstituted alkylene, substituted or unsubstituted arylene, or substituted or unsubstituted heteroarylene, n is 5 to 2000, and m is 0 to 1000.

15. A method for preparing a polyimide, comprising imidizing the polyimide acid according to any one of claims 9 and 10 or the polyimide acid prepared by the preparation method according to claim 11, to obtain the polyimide, the polyimide comprising a repeat unit represented by Formula (VI), wherein R₁ and R₂ are independently selected from a single bond, substituted or unsubstituted alkylene, substituted or unsubstituted alkenylene, substituted or unsubstituted alkynylene, substituted or unsubstituted arylene, substituted or unsubstituted arylenealkyl, substituted or unsubstituted heteroarylene, substituted or unsubstituted heteroarylenealkyl, or substituted or unsubstituted alicylidene, and R₇ is selected from a substituted or unsubstituted arylene or substituted or unsubstituted heteroarylene,

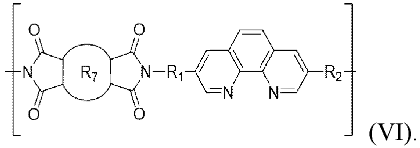

16. A battery assembly (10), comprising a heat dissipation component (13), a polyimide film (12) arranged on a surface of the heat dissipation component (13), and a battery core (11) arranged on a surface of the polyimide film (12), the polyimide film (12) being made of the polyimide according to claim 12 or the polyimide prepared by the preparation method according to claim 15.

17. The battery assembly (10) according to claim 16, wherein the surface of the heat dissipation component (13) is of a metal material, and the surface of the battery core (11) is of a metal material.

18. An electronic device, comprising a battery assembly (10) according to claim 16 or 17.
